# EUROPEAN PATENT SPECIFICATION

(11) **EP 2 991 649 B1**
(45) Date of publication and mention of the grant of the patent: **03.06.2020**
(21) Application number: 14791196.0
(22) Date of filing: 30.04.2014
(51) Int. Cl.: A61K 31/437, A61K 31/427, A61K 31/7036, A61K 31/7042, A61K 45/06, A61K 38/14, A61K 9/00, A61K 9/28, A61P 25/00, A61P 43/00, A23L 33/10

(54) **COMPOSITIONS FOR TREATING OBSESSIVE-COMPULSIVE DISORDER**
ZUSAMMENSETZUNGEN ZUR BEHANDLUNG DER ZWANGSSTÖRUNG
COMPOSITIONS DESTINÉES AU TRAITEMENT DU TROUBLE OBSESSIONNEL COMPULSIF

(30) Priority: 30.04.2013 US 201361817769 P
(43) Date of publication of application: 09.03.2016
(73) Proprietor: Borody, Thomas Julius, Five Dock, NSW 2046 (AU)
(72) Inventor: Borody, Thomas Julius, Five Dock, NSW 2046 (AU)
(74) Representative: Zwicker, Jörk
(86) International application number: PCT/AU2014/000478
(87) International publication number: WO 2014/176632

(56) References cited:
- WO-A1-02/07741
- WO-A1-2009/137672
- WO-A1-2010/040020
- WO-A1-2011/050397
- WO-A1-2014/032108
- CA-A1- 2 391 422
- US-A1- 2012 252 775
- HEBERLING COLIN A ET AL: "Hypothesis for a systems connectivity model of autism spectrum disorder pathogenesis: Links to gut bacteria, oxidative stress, and intestinal permeability", MEDICAL HYPOTHESES, EDEN PRESS, PENRITH, US, vol. 80, no. 3, 27 December 2012 (2012-12-27), pages 264-270, XP028979869, ISSN: 0306-9877, DOI: 10.1016/J.MEHY.2012.11.044
- KIM, Y.-H. ET AL.: 'Effect of Rifampin on the Plasma Concentration and the Clinical Effect of Haloperidol Concomitantly Administered to Schizophrenic Patients' JOURNAL OF CLINICAL PSYCHOPHARMACOLOGY vol. 16, no. ISSUE, June 1996, pages 247 - 252, XP008181385
- TAKEDA, M. ET AL.: 'Serum Haloperidol Levels of Schizophrenics Receiving Treatment for Tuberculosis' CLINICAL NEUROPHARMACOLOGY vol. 9, no. 4, August 1986, pages 386 - 397, XP008181380

## Description

### TECHNICAL FIELD

This invention generally relates to compositions for use in treating, ameliorating and preventing obsessive compulsive disorder in mammals where the microbial or bacterial flora of the bowel is at least one causative or symptom-producing factor, for example, where the microbial or bacterial flora of the bowel manufactures neurotoxins or neurotoxic agents that enter the body through the gastrointestinal (GI) tract, e.g. the colon, and reach the systemic space, e.g., by neural streaming or via the circulation, to reach the central nervous system (CNS), including the brain, the peripheral nervous system (PNS), and other nervous systems.

### BACKGROUND

Until recent years most mental disorders including obsessive compulsive disorder (OCD) have been, and continue to be, considered as being caused by psychological or psychiatric aberrations. Sigmund Freud indicated that OCD behaviour was due to unconscious conflicts that later manifested as symptoms. He reached back into early childhood experiences where the 'need to touch' which was externally prohibited by parents created an OCD type behaviour. Most OCD therapy is currently psychological therapy, but it can also use drugs to suppress certain neurotransmitter activity within the brain.

Obsessive compulsive disorders are classified under 'anxiety disorders' where the patient has a thinking process which produces uneasiness, apprehension, worry, fear, repetitive behaviours all aimed at reducing the anxiety. The obsessions can be associated with compulsions. The symptoms can include repetitive religious thoughts, aversion to particular numbers, nervous rituals, opening closing doors, excessive washing or cleaning, repeated checking, extreme hoarding movements of arms or legs or entering and leaving a room. These symptoms interfere with life and can alienate friends and relatives. They can cause severe emotional and financial distress. However, OCD sufferers do recognize their obsessions and compulsions and they realise they are irrational. This further distresses them. More than 80% of these begin in childhood and there is a certain amount of cross over between OCD and other disorders which share such behaviour, including autism spectrum disorder, ADHD, PTSD and profound anxiety.

Patients with OCD do not have below-average intelligence and OCD is actually associated with a higher IQ. OCD syndrome can also co-exist with major depressive disorders, bipolar disorders, anorexia nervosa, bulimia, generalised anxiety disorder, Tourrets' syndrome, Asperger's syndrome, Attention Deficit Hyperactivity Disorder. Dermatillomania, Trichotillomania as well as body dismorphic disorder can be associated with OCD. Delayed sleep may be also a feature but particularly depression is an extremely common syndrome among OCD patients. In around 80% of the cases symptoms present before the age of 18 with 1 to 3% of the population suffering with this disorder. It is estimated that in the US lifetime prevalence of OCD is 2.5%. Some estimates also say that around 2.2 million US residents have or have had OCD. Other countries have similar rates.

Patients with this disability with its associated co-morbidities, are therefore looking for some advance in therapy rather than continuing with the partially-effective but never curative psychological intervention such as behavioural and cognitive/behavioural therapies. Pharmacological treatment can lead to substantial reduction in symptoms but they do keep on persisting at moderate levels throughout life. To be completely symptom free is uncommon.

Children with autism have been noted to possess abnormal *Clostridia* and the *Desulfovibrio* bacteria in the stool, see e.g., Finegold (2012) Anaerobe 18:260.

### SUMMARY

The scope of the present invention is defined in the appended claims.

The invention provides formulations, pharmaceuticals or pharmaceutical preparations comprising at least one active agent, wherein the active agent comprises:
a rifaximin, an extended intestinal release (EIR) rifaximin, a rifamycin derivatives selected from the goup consisting of a rifampicin (or rifampin), a rifabutin, a rifapentine and a rifalazil for use in treating, ameliorating and preventing obsessive compulsive disorder (OCD).

In one embodiment the formulation, a pharmaceutical or a pharmaceutical preparation is formulated as a chewable delivery vehicle, a gum, a candy, a lozenge, an ice cream or an ice, or a yogurt,

In one embodiment the unit dosage is a pediatric unit dosage, and optionally the unit dosage is between 10 mg and 1100 mg, or is 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 mg per unit dose.

In one embodiment the daily dosage is 100, 125, 150, 175, 200, 225, 250, 275, 300, 325, 350, 375, 400, 425, 450, 475, 500, 600, 700, 750, 800, 900, 1000 or 1100 mg per day, or between 100 and 1100 mg per day,

In one embodiment the unit dosage is set for bid (twice a day), tid (three times a day), four times a day, five times a day or six times a day or more, with the unit dosage and daily dosage adjusted to be: 1000 mg/70 kg a day, or 14 mg/kg a day, for an adult median dose per day (five times a day for adult use, or for a 70 kg individual would be 200 mg per unit dose); or for a pediatric dosage 350 mg/25 kg a day, or 15 to 16 mg/kg, a day; or equivalent (five times a day for pediatric use, or for a 25 kg individual, would be 70 mg per unit dose).

In one embodiment the formulation, pharmaceutical or pharmaceutical preparation comprises a combination of a rifaximin together with a vancomycin.

In alternative embodiments, the formulation, pharmaceutical or pharmaceutical preparation further comprises: a flavoring or a sweetening agent, an aspartamine, a stevia, monk fruit, a sucralose, a saccharin, a cyclamate, a xylitol, a vanilla, an artificial vanilla or chocolate or strawberry flavor, an artificial chocolate essence, or a mixture or combination thereof. In alternative embodiments, the formulation, pharmaceutical or pharmaceutical preparation further comprises: a preservative, a benzoic acid, a potassium sorbate.

In alternative embodiments, the formulation, pharmaceutical or pharmaceutical preparation further comprises or has added to: at least one probiotic or prebiotic, wherein optionally the prebiotic comprises an inulin, lactulose, extracts of artichoke, chicory root, oats, barley, various legumes, garlic, kale, beans or flacks or an herb, wherein optionally the probiotic comprises a cultured or stool-extracted microorganism or bacteria, or a bacterial component, and optionally the bacteria or bacterial component comprises or is derived from a *Bacteroidetes,* a *Firmicutes,* a *Lactobacilli,* a *Bifidobacteria,* an *E coli,* a *Strep fecalis* and equivalents.

In alternative embodiments, the formulation, pharmaceutical or pharmaceutical preparation further comprises or has added to: at least one congealing agent, wherein optionally the congealing agent comprises an arrowroot or a plant starch, a powdered flour, a powdered potato or potato starch, an absorbant polymer, an Absorbable Modified Polymer (AMP®), EndoClot, Santa Clara, CA), and/or a corn flour or a corn starch.

In alternative embodiments, the formulation, pharmaceutical or pharmaceutical preparation further comprises or has added to: at least one an anti-inflammatory agent, wherein optionally the inflammatory agent comprises or is a 4 or a 5-amino-salicylate, an olsalazine (e.g., DIPENTUM™), a mesalazine (also known as mesalamine or a 5-aminosalicylic acid (5-ASA), e.g., ASACOL™ or LIALDA™), a sulfasalazine (e.g., AZULFIDINE™, SALAZOPYRIN™ or SULAZINE™), and/or a balsalazide (e.g. COLAZAL™ or COLAZIDE™), or an equivalent thereof or a combination thereof.

In alternative embodiments, the formulation, pharmaceutical or pharmaceutical preparation further comprises or has added to: an additive selected from one or more of a saline, a defoaming agent, a surfactant agent, a lubricant, an acid neutralizer, a marker, a cell marker, a contrast agent, a dispersal agent, a buffer or a buffering agent, a debittering agent, a pH stabilizer, an acidifying agent, a desweetening agent and/or coloring agent, vitamin, mineral and/or dietary supplement, and/or a prebiotic nutrient.

In alternative embodiments, the formulation, pharmaceutical or pharmaceutical preparation further comprises or has added to: at least one Biofilm Disrupting Compound, wherein optionally the biofilm disrupting compound comprises an enzyme, a deoxyribonuclease (DNase), N-acetylcysteine, an auranofin, an alginate lyase, glycoside hydrolase dispersin B; a Quorum-sensing inhibitor, a ribonucleic acid III inhibiting peptide, *Salvadora persica* extracts, Competence-stimulating peptide, Patulin and penicillic acid; peptides - cathelicidin-derived peptides, small lytic peptide, PTP-7, Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

In alternative embodiments, the formulation, pharmaceutical or pharmaceutical preparation is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, such as EUDRAGIT S™ (Evonik Industries AG, Essen, Germany), which dissolves at pH 7 or greater, e.g., comprises a multimatrix (MMX) formulation.

In alternative embodiments, the formulation, pharmaceutical or pharmaceutical preparation further comprises or has added to: an additional antimicrobial or antibiotic, wherein optionally the additional antimicrobial or antibiotic comprises: an ampicillin, a sulbactama tetracycline, a cephalosporin, a carbapenem, an imipenem, a meropenem, a MONAN™, MERONEM™, a monobactam, a lincosamide, a clindamycin, a DALACIN™, a quinolone, a fluoroquinolone, a sulphonamide, a fradicin, a NEOBIOTIC™, a nitroimidazole, a metronidazole, a tinidazole, an anti-clostridial agent, or a ramoplanan, an aminoglycoside antibiotic, a gentamycin, a neomycin, a streptomycin, a paromomycin, a verdamicin, a mutamicin, a sisomicin, a netilmicin, a retymicin, a kanamycin, an amphenicol, an ansamycin, a beta-lactam (β-lactam) antibiotic, a carbapenem, a cephalosporin, a cephamycin, a monobactam, an oxacephem, a lincosamide antibiotic, a clindamycin, or a lincomycin, a glycopeptide antibiotic, a vancomycin, a teicoplanin, a telavancin, a bleomycin, a ramoplanin, a decaplanin, a polypeptide antibiotic, an actinomycin, an actinomycin D, a bacitracin, a bacitracin, a tetracycline, a 2,4-diaminopyrimidine class antibiotic, a clavacin, a clairformin, a claviform, an expansine, a clavatin, an expansin, a gigantin, a leucopin, a patuline or a patulin), or an equivalent thereof or a combination thereof.

Also disclosed are a delivery vehicle, a product of manufacture, a container, a syringe, a device or a bag or device, comprising: a formulation, a pharmaceutical or a pharmaceutical preparation of the invention.

Also disclosed are a delivery vehicle, a product of manufacture, a container, a syringe, a device or a bag or device comprising: a formulation, a pharmaceutical or a pharmaceutical preparation of the invention, initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

The details of one or more embodiments of the invention are set forth in the accompanying drawings and the description below. Other features, objects, and advantages of the invention will be apparent from the description and drawings, and from the claims.

Reference will now be made in detail to various exemplary embodiments of the invention, examples of which are illustrated in the accompanying drawings. The following detailed description is provided to give the reader a better understanding of certain details of aspects and embodiments of invention.

### DETAILED DESCRIPTION

The invention provides formulations and pharmaceutical preparations for use in treating, ameliorating and preventing obsessive compulsive disorder (OCD) in mammals where the microbial or bacterial flora of the bowel is at least one causative or symptom-producing factor, for example, where the microbial or bacterial flora of the bowel manufactures neurotoxins or neurotoxic agents that enter the body through the gastrointestinal (GI) tract, e.g. the colon, and reach the systemic space, e.g., by neural streaming or via the circulation, to reach the central nervous system (CNS), including the brain, the peripheral nervous system (PNS), and other nervous systems.

The formulations, pharmaceuticals or pharmaceutical preparations of the invention may be formulated in delivery forms especially applicable to children, and/or for long-term use, particularly for children. In alternative embodiments, compositions of the invention, can take the form of a capsule, a geltab, a pill, a dissolvable wafer, a tablet, a chewable sweet, a lolly (lollypop), a lozenge or a candy, or as a smoothy or a jelly, or ices, ice creams, gelatos, yogurts or drinks.

Delivery forms e.g., a yogurt or a drink, may be designed such that the solid component of the rifaximin, polymorphic form or analog thereof, or equivalent thereof, is kept without degradation inside a separate sealed space which can be emptied into the delivery vehicle, e.g., drink or yogurt, in a twist top which will release granules of the active agent (e.g., comprising the rifaximin, polymorphic form or analog thereof, or equivalent thereof, or mixtures thereof) into the drink, or yogurt or the like, and then dissolves and is eaten or drunk by the child.

The formulations, pharmaceuticals or pharmaceutical preparations of the invention may be enterically coated, e.g., in an MMX enteric extend release format, such that active agent (e.g., comprising the rifaximin, polymorphic form or analog thereof, or equivalent thereof, or mixtures thereof) is delivered throughout the small through to the large bowel to affect its activity on the desired microbe, e.g., the pathogen that causes OCD.

The formulation, pharmaceutical or pharmaceutical preparation of the invention may be a flavoured chewable tablet, sweet or candy which the child is directed to take, e.g., more than once a day, e.g., twice or three times daily, to maintain suppression of the OCD producing agents in the flora.

Other forms of administration are also contemplated such as tablets, capsules, liquids, lyophilized forms, creams, ointments, gels, powders and pastes.

### Additional or Optional Ingredients

The formulations, pharmaceuticals or pharmaceutical preparations of the invention may further comprise other non-absorbable antibiotics or medications can be used in addition rifaximin, or rifampicin or equivalents as disclosed in the claims.

In alternative embodiments, rifaximin can also be combined with other active agents or medications in the situation where the causative agent, e.g., the pathogenic microbe, e.g., an OCD pathogen, has some resistance to the rifaximin. For example, in alternative embodiments, rifamixin is combined with vancomycin.

### Antibiotics, Antimicrobials

Other non-absorbable medications may be used in addition to rifaximin; for example, a vancomycin, a streptomycin, a fidaxomicin, a gentamicin, a kanamycin, an amikacin, an arbekacin, a neomycin, a netilmicin, a paromomycin, rhodostreptomycin, a tobramycin, an apramycin and mixtures thereof. In alternative embodiments, Ampicillin, Sulbactam or Aztreonam macrolides, e.g., Clarithromycin and Azithromycin can also be used. In alternative embodiments, Nitroimidazoles, such as Metronidazole, Tinidazole can also be employed. In alternative embodiments, other anti-clostridial agents such as Ramoplanan can also be combined with rifaximin.

Doses (unit dosage forms) of rifaximin, polymorphic forms or equivalents thereof, or vancomycin, can be from between 10 mg and 10 g or between 100 mg and 500 mg, or 10, 20, 30,40, 50, 75,100, 200, 300,400, 500 or 1000 mg. Dosages can be adjusted depending on the combination of active agents used, particularly when using two or three or more combination drugs. For example, in alternative embodiments, combination used to practice the compositions or methods of the invention include: streptomycin, Gentamicin, Kanamycin and mixtures thereof; Ampicillin, Sulbactam or Aztreonam and various macrolides, e.g., Clarithromycin; Nitroimidazoles such as Metronidazole; Tinidazole; any anti-clostridial agents such as a Ramoplanan; and/or a Fidaxomicin.

The formulation, pharmaceutical or pharmaceutical preparation of the invention may further comprise added secondary medications to bolster its activity. These comprise rifaximin with vancomycin, or the combination of Rifaximin with Astreonam or Rifaximin with Astreonam together with beta-lactamase inhibitors. In alternative embodiments these inhibitors are Clavulenic acid, Tazobactam, Sulbactam, LK-157 or equivalents.

The formulations, pharmaceuticals or pharmaceutical preparations of the invention may be combined in double or triple therapy format; including, for example, an astreonam, a streptomycin, a gentamicin, a kanamycin or a macrolides, or any combination thereof. In alternative embodiments metronidazole is also added to ensure that any neural streaming toxins where the bacteria reside in the submucosa, is also treated.

In alternative embodiments, antibiotics and/or other antimicrobials are included in a composition of the invention, e.g., added back to a liquid formulation or preparation of the invention, or cell preparation of the invention. In alternative embodiments, the antimicrobial or antibiotic is or comprises one or more of a: glycopeptide antibiotic, wherein optionally the glycopeptide antibiotic is a vancomycin, a teicoplanin (e.g., TARGOCID™), a telavancin (e.g., VIBATIV™), a bleomycin (e.g., BLENOXANE™), a ramoplanin or a decaplanin; or, a fidaxomycin, a gentamycin, a verdamicin, a mutamicin, a sisomicin, a netilmicin, a retymicin, a neomycin, a streptomycin, a paromomycin, a kanamycin, or an ansamycin, a geldanamycin, an ansamitocin, or an anti-protozoal agent such as nitazoxanide (e.g., DAXON™, DEXIDEX™, KIDONAX™, MITAFAR™, PACOVANTON™, PARAMIX™), a furazolidone (e.g., FUROXONE™, DEPENDAL-M™), a nitroimidazole or metronidazole (e.g., a 5-nitroimidazole, FLAGYL™), a nifuroxazide (e.g., AMBATROL™, ANTINAL™, BACIFURANE™, DIAFURYL™) or a bismuth (e.g., bismuth subsalicylate), also including various groups of antibiotics such as a penicillin (e.g., penicillin G, procaine penicillin, benzathine penicillin or penicillin V), a macrolide (e.g., erythromycin, a clarithromycin (a TRUCLAR™, CRIXAN™, CLARITT™, CLARAC™, BIAXIN™, KLARICID™, KLACID™, KLARAM™, KLABAX™, CLARIPEN™, CLAREM™, CLARIDAR™, FROMILID™, CLACID™, CLACEE™, VIKROL™, INFEX™, CLARIWIN™, RESCLAR™, RANBAXY™ or a CLARIHEXAL™), a dirithromycin (e.g., DYNABAC™), a roxithromycin (e.g., XTHROCIN™, ROXL-150™, ROXO™, SURLID™), a telithromycin (e.g., KETEK™) or an azithromycin such a ZITHROMAX™, AZITHROCIN™), a tetracycline, a cephalosporin, a carbapenem (e.g., imipenem, a meropenem such as MONAN™, MERONEM™), a monobactam, a lincosamide or a clindamycin (e.g., DALACIN™), a quinolone (e.g., a fluoroquinolone) and/or a sulphonamide, a fradicin (e.g., NEOBIOTIC™), or an equivalent thereof or a combination thereof.

In alternative embodiments, the antimicrobial or antibiotic is or comprises one or more of: an aminoglycoside antibiotic (e.g., a gentamycin, a neomycin, a streptomycin, a paromomycin, a verdamicin, a mutamicin, a sisomicin, a netilmicin, a retymicin and/or a kanamycin), amphenicol, ansamycin, beta-lactam (β-lactam), carbapenem, cephalosporin, cephamycin, monobactam, oxacephem, a lincosamide antibiotic (e.g., clindamycin, lincomycin), a macrolide antibiotic (e.g., an azithromycin, clarithromycin, dirithromycin, erythromycin), glycopeptide antibiotic (e.g., a vancomycin, teicoplanin, telavancin, bleomycin, ramoplanin and/or a decaplanin), a polypeptide antibiotic (e.g., actinomycin, such as actinomycin D; bacitracin; bacitracin), tetracycline, or a 2,4-diaminopyrimidine class antibiotic, a clavacin (also known as clairformin, claviform, expansine, clavatin, expansin, gigantin, leucopin, patuline or patulin), or an equivalent thereof or a combination thereof.

### Probiotics and prebiotics

In alternative embodiments, additives that are also included in a composition of the invention (e.g., a liquid preparation embodiment), or a composition used to practice the invention, includes one or more prebiotics such as inulin, lactulose, extracts of artichoke, chicory root, oats, barley, various legumes, garlic, kale, beans or flacks and at times prebiotics may include herbs.

In alternative embodiments, additives may include flora components such as *Bacteroidetes, Firmicutes, Bacillus* (e.g., *Bacillus thurigiensis*) or any combination thereof. In alternative embodiments, cultured components are back to the flora to fortify or expand specific genus or species, e.g., *Bacteroidetes, Firmicutes, Bacillus* or *Bacillus thurigiensis.* Probiotics may at times be included as single cultured components. They would avoid multiply cultured components as they lose their implantation characteristics.

### Preservatives, Cryoprotectants, Lyoprotectants

In alternative embodiments, to any composition of the invention (e.g., the liquid preparation embodiment, candies, lollies, drinks and the like) may be added various preservatives, cryoprotectants and/or lyoprotectants, including e.g., various polysaccharides or sugars (such as sucrose, fructose, lactose, mannitol), glycerol, polyethylene glycol (PEG), trehalose, glycine, glucose, dextran and/or erythritol. In alternative embodiments, other cryoprotectants that can be used are ethylene glycol, 1,2-Propanediol, Methylcelliosolve, Dimethyl Formamide, or Dimethylsulphoxide Methanol. In alternative embodiments the content of these cryoprotectants are between about 1% and about 50% but generally between about 5% and about 15% is adequate.

Because of the ability to freeze and/or freeze-dry, or spray dry, any composition of the invention, in alternative embodiments there are different types of final products that can be manufactured. In alternative embodiments a product or formulation of the invention is a liquid. In alternative embodiments a product or formulation of the invention is frozen and kept at e.g. minus 80 degrees for usage later given a cryoprotectant is added.

### Biofilm Disrupting Compounds

In alternative embodiments, biofilm disrupting compounds added into a composition or formulation of the invention (e.g., a liquid preparation). Biofilm disrupting compounds may be administered before or during (co-administered), or co-formulated with (e.g., in a multilaminated tablet or capsule), or separately formulated, as the administered composition or formulation of the invention. Distrupting biofilms may be used to separate from the colonic mucosa an adherent polysaccharide/DNA - containing layer, the so-called "biofilm.

Other biofilm disrupting components or agents also can be used, e.g., enzymes such as a deoxyribonuclease (DNase), a N-acetylcysteine, an auranofin, alginate lyase, glycoside hydrolase dispersin B; Quorum-sensing inhibitors e.g., ribonucleic acid III inhibiting peptide, *Salvadora persica* extracts, Competence-stimulating peptide, Patulin and penicillic acid; peptides - cathelicidin-derived peptides, small lytic peptide, PTP-7 (a small lytic peptide, see e.g., Kharidia (2011) J. Microbiol. 49(4):663-8, Epub 2011 Sep 2), Nitric oxide, neo-emulsions; ozone, lytic bacteriophages, lactoferrin, xylitol hydrogel, synthetic iron chelators, cranberry components, curcumin, silver nanoparticles, Acetyl-11-keto-β-boswellic acid (AKBA), barley coffee components, probiotics, sinefungin, S-adenosylmethionine, S-adenosyl-homocysteine, Delisea furanones, N-sulfonyl homoserine lactones and/or macrolide antibiotics or any combination thereof.

Biofilm disrupting components or agents may be administered before and during the administration of a composition of this invention, e.g., as an FMT, in whatever format or formulation this may take place, for example, as a capsule.

Biofilm disrupting agents may be added either before treatment and/or during and/or after treatment with a composition of the invention. Biofilm disrupting agents are used singly or in combination.

Biofilm disrupting agents include particular enzymes and degrading substances including in N-acetylcysteine, deoxyribonuclease (DNase). Others would include Alginate, lyase and Glycoside hydrolase dispersin, Ribonucleic-acid-III inhibiting peptide (RIP), Salvadora persica extracts, Competence-stimulating peptide (CSP) Patulin (PAT) and penicillic acid (PA)/EDTA, Cathelicidin-derived peptides, Small lytic peptide, PTP-7, Nitric oxide, Chlorhexidine, Povidone-iodine (PI), Nanoemulsions, Lytic bacteriophages, Lactoferrin/xylitol hydrogel, Synthetic iron chelators, Cranberry components, Curcumin, Acetyl-11-keto-boswellic acid (AKBA), Barley coffee (BC) components, silver nanoparticles, azithromycin, clarithromycin, gentamicin, streptomycin and also Disodium EDTA. Ozone insufflations of the colon can also be used to disrupt the biofilm.

### Unit dosage forms and formulations, foods, and delivery vehicles

A composition of the invention (e.g., a liquid preparation embodiment) can be further processed by, e.g., spray-drying or equivalent, e.g., spray-drying in an inert gas or freeze-drying under similar conditions, thus ending up with a powdered product. In alternative embodiments, a composition is manufactured, labelled or formulated as a liquid, a suspension, a spray, a gel, a geltab, a semisolid, a tablet, or sachet, a capsule, a lozenge, a chewable or suckable unit dosage form, or any pharmaceutically acceptable formulation or preparation. A composition of the invention may be incorporated into a food or a drink (e.g., a yogurt, ice cream, smoothie), a candy, sweet or lolly, or a feed, a nutritional or a food or feed supplement (e.g., liquid, semisolid or solid).

For example, a composition of the invention can be manufactured, labelled or formulated as an orally disintegrating tablet as described e.g., in U.S. Pat. App. Publication No. 20100297031. A composition of the invention can be a polyol/thickened oil suspension as described in U.S. Pat. No. (USPN) 6,979,674; 6,245,740. A composition of the invention can be encapsulated, e.g., encapsulated in a glassy matrix as described e.g., in U.S. Pat. App. Publication No. 20100289164; and USPN 7,799,341. A composition of the invention can be manufactured, labeled or formulated as an excipient particle, e.g., comprising a cellulosic material such as microcrystalline cellulose in intimate association with silicon dioxide, a disintegrant and a polyol, sugar or a polyol/sugar blend as described e.g., in U.S. Pat. App. Publication No. 20100285164. A composition of the invention can be manufactured, labeled or formulated as an orally disintegrating tablet as described e.g., in U.S. Pat. App. Publication No. 20100278930. A composition of the invention can be manufactured, labeled or formulated as a spherical particle, as described e.g., in U.S. Pat. App. Publication No. 20100247665, e.g., comprising a crystalline cellulose and/or powdered cellulose. A composition of the invention can be manufactured, labeled or formulated as a rapidly disintegrating solid preparation useful e.g. as an orally-disintegrating solid preparation, as described e.g., in U.S. Pat. App. Publication No. 20100233278. A composition of the invention can be manufactured, labeled or formulated as a solid preparation for oral application comprising a gum tragacanth and a polyphosphoric acid or salt thereof, as described e.g., in U.S. Pat. App. Publication No. 20100226866.

A composition of the invention can be manufactured, labeled or formulated using a water soluble polyhydroxy compound, hydroxy carboxylic acid and/or polyhydroxy carboxylic acid, as described e.g., in U.S. Pat. App. Publication No. 20100222311. A composition of the invention can be manufactured, labeled or formulated as a lozenge, or a chewable and suckable tablet or other unit dosage form, as described e.g., in U.S. Pat. App. Publication No. 20100184785.

A composition of the invention can be manufactured, labeled or formulated in the form of an agglomerate, as described e.g., in U.S. Pat. App. Publication No. 20100178349. A composition of the invention can be manufactured, labeled or formulated in the form of a gel or paste, as described e.g., in U.S. Pat. App. Publication No. 20060275223. A composition of the invention can be manufactured, labeled or formulated in the form of a soft capsule, as described e.g., in USPN 7,846,475, or USPN 7,763,276.

The polyols used in compositions of the invention can be micronized polyols, e.g., micronized polyols, e.g., as described e.g., in U.S. Pat. App. Publication No. 20100255307, e.g., having a particle size distribution (d₅₀) of from 20 to 60 µm, and a flowability below or equal to 5 s/100 g, or below 5 s/100 g.

### Gradual or Delayed Release Formulations

In alternative embodiments, the invention provides compositions formulated for delayed or gradual enteric release comprising at least one active agent (e.g., a formulation or pharmaceutical preparation of the invention) formulated with a delayed release composition or formulation, coating or encapsulation. In alternative embodiments, formulations or pharmaceutical preparations of the invention are designed or formulated for delivery of active ingredient (e.g., a rifaximin or a rifaximin in its various molecular versions or polymorphic forms, including for example, the extended intestinal release (EIR) rifaximin, or another rifamycin such as e.g., the rifamycin derivatives rifampicin (or rifampin), rifabutin, rifapentine and/or rifalazil, or XIFAXAN™) into the distal small bowel and/or the colon. Thus, for this embodiment, it is important to allow the active ingredient to pass the areas of danger, e.g., stomach acid and pancreatic enzymes and bile, and reach undamaged to be viable in the distal small bowel and especially the colon. In alternative embodiments, a formulation or pharmaceutical preparation of the invention is a liquid formulation, a microbiota-comprising formulation of the invention and/or a frozen or a freeze-dried version thereof. In alternative embodiments, preferably for the encapsulated format, all are in powdered form.

In alternative embodiments, compositions of the invention are formulated for delayed or gradual enteric release using cellulose acetate (CA) and polyethylene glycol (PEG), e.g., as described by Defang et al. (2005) Drug Develop. & Indust. Pharm. 31:677-685, who used CA and PEG with sodium carbonate in a wet granulation production process.

In alternative embodiments, compositions of the invention are formulated for delayed or gradual enteric release using a hydroxypropylmethylcellulose (HPMC), a microcrystalline cellulose (MCC) and magnesium stearate, as described e.g., in Huang et al. (2004) European J. of Pharm. & Biopharm. 58: 607-614).

In alternative embodiments, compositions of the invention are formulated for delayed or gradual enteric release using e.g., a poly(meth)acrylate, e.g. a methacrylic acid copolymer B, a methyl methacrylate and/or a methacrylic acid ester, a polyvinylpyrrolidone (PVP) or a PVP-K90 and a EUDRAGIT® RL PO™, as described e.g., in Kuksal et al. (2006) AAPS Pharm. 7(1), article 1, E1 to E9.

In alternative embodiments, compositions of the invention are formulated for delayed or gradual enteric release as described in U.S. Pat. App. Pub. 20100239667. In alternative embodiments, the composition comprises a solid inner layer sandwiched between two outer layers. The solid inner layer can comprise a formulation or pharmaceutical preparation of the invention and one or more disintegrants and/or exploding agents, one of more effervescent agents or a mixture. Each outer layer can comprise a substantially water soluble and/or crystalline polymer or a mixture of substantially water soluble and/or crystalline polymers, e.g., a polyglycol. These can be adjusted in an exemplary composition of the invention to achieve delivery of the living components of an FMT distally down the bowel.

In alternative embodiments, compositions of the invention are formulated for delayed or gradual enteric release as described in U.S. Pat. App. Pub. 20120183612, which describes stable pharmaceutical formulations comprising active agents in a non-swellable diffusion matrix. In alternative embodiments, a formulation or pharmaceutical preparation of the invention is released from a matrix in a sustained, invariant and, if several active agents are present, independent manner and the matrix is determined with respect to its substantial release characteristics by ethylcellulose and at least one fatty alcohol to deliver bacteria distally.

In alternative embodiments, a formulation or pharmaceutical preparation of the invention is formulated for delayed or gradual enteric release as described in U.S. Pat. No. 6,284,274, which describes a bilayer tablet containing an active agent (e.g., an opiate analgesic), a polyalkylene oxide, a polyvinylpyrrolidone and a lubricant in the first layer and a second osmotic push layer containing polyethylene oxide or carboxymethylcellulose.

In alternative embodiments, a formulation or pharmaceutical preparation of the invention is formulated for delayed or gradual enteric release as described in U.S. Pat. App. Pub. No. 20030092724, which describes sustained release dosage forms in which a nonopioid analgesic and opioid analgesic are combined in a sustained release layer and in an immediate release layer, sustained release formulations comprising microcrystalline cellulose, EUDRAGIT RSPO™, CAB-O-SIL™, sodium lauryl sulfate, povidone and magnesium stearate.

In alternative embodiments, a formulation or pharmaceutical preparation of the invention is formulated for delayed or gradual enteric release as described in U.S. Pat. App. Pub. 20080299197, describing a multi-layered tablet for a triple combination release of active agents to an environment of use, e.g., in the GI tract. In alternative embodiments, a multi-layered tablet is used, and it can comprise two external drug-containing layers in stacked arrangement with respect to and on opposite sides of an oral dosage form that provides a triple combination release of at least one active agent. In one embodiment the dosage form is an osmotic device, or a gastro-resistant coated core, or a matrix tablet, or a hard capsule. In these alternative embodiments, the external layers may contain biofilm dissolving agents and internal layers the living bacteria.

In alternative embodiments, a formulation or pharmaceutical preparation of the invention is formulated as multiple layer tablet forms, e.g., where a first layer provides an immediate release of a formulation or pharmaceutical preparation of the invention and a second layer provides a controlled-release of another (or the same) formulation or pharmaceutical preparation of the invention, or another active agent, as described e.g., in U.S. Pat. No. 6,514,531 (disclosing a coated trilayer immediate/prolonged release tablet), U.S. Pat. No. 6,087,386 (disclosing a trilayer tablet), U.S. Pat. No. 5,213,807 (disclosing an oral trilayer tablet with a core comprising an active agent and an intermediate coating comprising a substantially impervious/impermeable material to the passage of the first active agent), and U.S. Pat. No. 6,926,907 (disclosing a trilayer tablet that separates a first active agent contained in a film coat from a core comprising a controlled-release second active agent formulated using excipients which control the drug release, the film coat can be an enteric coating configured to delay the release of the active agent until the dosage form reaches an environment where the pH is above four).

In alternative embodiments, a formulation or pharmaceutical preparation of the invention is formulated for delayed or gradual enteric release as described in U.S. Pat. App. Pub. 20120064133, which describes a release-retarding matrix material such as: an acrylic polymer, a cellulose, a wax, a fatty acid, shellac, zein, hydrogenated vegetable oil, hydrogenated castor oil, polyvinylpyrrolidine, a vinyl acetate copolymer, a vinyl alcohol copolymer, polyethylene oxide, an acrylic acid and methacrylic acid copolymer, a methyl methacrylate copolymer, an ethoxyethyl methacrylate polymer, a cyanoethyl methacrylate polymer, an aminoalkyl methacrylate copolymer, a poly(acrylic acid), a poly(methacrylic acid), a methacrylic acid alkylamide copolymer, a poly(methyl methacrylate), a poly(methacrylic acid anhydride), a methyl methacrylate polymer, a polymethacrylate, a poly(methyl methacrylate) copolymer, a polyacrylamide, an aminoalkyl methacrylate copolymer, a glycidyl methacrylate copolymer, a methyl cellulose, an ethylcellulose, a carboxymethylcellulose, a hydroxypropylmethylcellulose, a hydroxymethyl cellulose, a hydroxyethyl cellulose, a hydroxypropyl cellulose, a crosslinked sodium carboxymethylcellulose, a crosslinked hydroxypropylcellulose, a natural wax, a synthetic wax, a fatty alcohol, a fatty acid, a fatty acid ester, a fatty acid glyceride, a hydrogenated fat, a hydrocarbon wax, stearic acid, stearyl alcohol, beeswax, glycowax, castor wax, carnauba wax, a polylactic acid, polyglycolic acid, a co-polymer of lactic and glycolic acid, carboxymethyl starch, potassium methacrylate/divinylbenzene copolymer, crosslinked polyvinylpyrrolidone, polyvinylalcohols, polyvinylalcohol copolymers, polyethylene glycols, non-crosslinked polyvinylpyrrolidone, polyvinylacetates, polyvinylacetate copolymers or any combination. In alternative embodiments, spherical pellets are prepared using an extrusion/ spheronization technique, of which many are well known in the pharmaceutical art. The pellets can comprise one or more formulations or pharmaceutical preparations of the invention, e.g., the liquid preparation embodiment.

In alternative embodiments, a formulation or pharmaceutical preparation of the invention is formulated for delayed or gradual enteric release as described in U.S. Pat. App. Pub. 20110218216, which describes an extended release pharmaceutical composition for oral administration, and uses a hydrophilic polymer, a hydrophobic material and a hydrophobic polymer or a mixture thereof, with a microenvironment pH modifier. The hydrophobic polymer can be ethylcellulose, cellulose acetate, cellulose propionate, cellulose butyrate, methacrylic acid-acrylic acid copolymers or a mixture thereof. The hydrophilic polymer can be polyvinylpyrrolidone, hydroxypropylcellulose, methylcellulose, hydroxypropylmethyl cellulose, polyethylene oxide, acrylic acid copolymers or a mixture thereof. The hydrophobic material can be a hydrogenated vegetable oil, hydrogenated castor oil, carnauba wax, candellia wax, beeswax, paraffin wax, stearic acid, glyceryl behenate, cetyl alcohol, cetostearyl alcohol or and a mixture thereof. The microenvironment pH modifier can be an inorganic acid, an amino acid, an organic acid or a mixture thereof. Alternatively, the microenvironment pH modifier can be lauric acid, myristic acid, acetic acid, benzoic acid, palmitic acid, stearic acid, oxalic acid, malonic acid, succinic acid, adipic acid, sebacic acid, fumaric acid, maleic acid; glycolic acid, lactic acid, malic acid, tartaric acid, citric acid, sodium dihydrogen citrate, gluconic acid, a salicylic acid, tosylic acid, mesylic acid or malic acid or a mixture thereof.

In alternative embodiments, a formulation or pharmaceutical preparation of the invention is a powder that can be included into a tablet or a suppository. In alternative embodiments, a formulation or pharmaceutical preparation of the invention can be a 'powder for reconstitution' as a liquid to be drunk placed down a naso-duodenal tube or used as an enema for patients to take home self-administer enemas for colitis for example. In alternative embodiments, a formulation or pharmaceutical preparation of the invention is micro-encapsulated, formed into tablets and/or placed into capsules, especially enteric-coated capsules. In alternative embodiments, in practicing the methods of the invention, biofilm disrupting compounds are administered before or during (co-administered), or co-formulated with a composition or formulation of the invention. For example, in alternative embodiments, a composition or formulation of the invention and a biofilm disrupting compound (and/or any other alternative component of the invention, as discussed herein) are co-formulated, e.g., as multiple layer tablet form or as a multi-laminated tablet or capsule. In alternative embodiments of methods of the invention, biofilm disrupting compounds are separately formulated.

### Feeds, drinks, candies, nutritional or a food or feed supplements

A formulation or pharmaceutical preparation of the invention may be incorporated into a food, a feed, a candy (e.g., a lollypop or a lozenge) a drink, a nutritional or a food or feed supplement (e.g., liquid, semisolid or solid), as described e.g., in U.S. Pat. App. Publication No. 20100178413. A formulation or pharmaceutical preparation of the invention may be incorporated into (manufactured as) a beverage as described e.g., in USPN 7,815,956. For example, a composition of the invention is incorporated into a yogurt, an ice cream, a milk or milkshake, a "frosty", "snow-cone", or other ice-based mix.

A formulation or pharmaceutical preparation of the invention may be a freeze-dried powder form added to a food, e.g., a yogurt, an ice cream, a milk or milkshake, a "frosty", "snow-cone", or other ice-based mix. In one form of this invention it can be kept in a lid-storage (e.g., of a yogurt or ice cream) such that when it is twisted the powder falls into the product or formulation (e.g., yoghurt or ice cream) and then it can be stirred so as not to have the powder ferment 'standing on the shelf'. Various flavourings can be added. In alternative embodiments, this is particularly important for administration of a composition of the invention, e.g., a wild type microbiota or a cultured bacteria, to a very young individual.

These exemplary products are important when administered to children or babies who may have acquired various pathogenic or abnormal bacteria, e.g., *E. coli, Clostridia* or *Disulfovibrio*.

### Applications of compositions of the invention

A formulation or pharmaceutical preparation of the invention may be used to treat, ameliorate, prevent or reverse: a obsessive compulsive disorder (OCD), where the microbial or bacterial flora of the bowel is at least one causative or symptom-producing factor, for example, where the microbial or bacterial flora of the bowel manufactures neurotoxins or neurotoxic agents that enter the body through the gastrointestinal (GI) tract, e.g. the colon, and reach the systemic space, e.g., by neural streaming or via the circulation, to reach the central nervous system (CNS), including the brain, the peripheral nervous system (PNS), and other nervous systems disorders and conditions.

The invention will be further described with reference to the following examples.

### EXAMPLES

### EXAMPLE 1: Exemplary treatment of OCD in adult with rifaximin

A 43year old female married office worker had suffered with OCD since childhood. Having been treated with various pharmacologic agents through life her behaviour patterns were largely controlled except for one behaviour of needing to check gas outlets and powerpoints every night in case her children were hurt or damaged overnight by some unforseen accident. This continued to be a 'sleep-draining' issue not controllable by Zoloft, anfranil and other drugs.

She was commenced on rifaximin 500 mg capsules twice daily for the first week, then 1g twice daily thereafter.

Within 10 days she noticed an absence of the need to check the gas outlets and powerpoints and was able to retire sleep through the night, perhaps first time in many years. This improvement was almost universal for the next 3 months of monitoring her behaviour. She remains on long term rifaximin.

### EXAMPLE 2: Exemplary treatment of OCD of child with rifaximin and vancomycin

A 13 year old male child was completely well until an overseas trip and a diarrhoeal illness which seemed to last 2-3 weeks. Seemingly unrelated he developed rather quickly, over several months patterns of progressively severe, repetitive behavioural changes diagnosed as OCD. This was managed by pharmacologic agents in a leading Institute for Anxiety Disorders. Drugs included fluoxetine and escitalopram. He had a documented number of some 56 repetitive behavioural patterns documented by the Institute.

He was commenced on increasing dose of vancomycin 250 mg bid and rifaxamin 550 mg bid. After four weeks of treatment there was already an improvement with a measurable reduction of the OCD pattern. From being unable to attend school nor see his school peers he was able to invite friends over as the symptoms were much less intrusive.

The dosage of the medications were raised to 500 mg bid of vancomycin and 2.2 g of rifaxamin per day in a bid dose. Over the next 12 months of treatment the repetitive behavioural groupings were reduced to 2 per day and he was able to cut down on his pharmacological treatment. He returned to school where he again reached the same level of excellence prior to developing his OCD syndrome.

He continues on the same dosages of vancomycin and rifaxamin 22 months later. Fluoxetine has now been stopped.

### EXAMPLE 3

Twin sisters who were diagnosed with Autism Spectrum Disorder (ASD) at different levels of reading capability, writing and comprehension in Grade 3 and 4, together with abdominal cramping and alternating hard/soft defecation changes were started on oral Gentamicin 80 mg 3 times daily in capsules. Each had a component of OCD in her psychotropic disorder but within three weeks the use of the Gentamicin had helped the OCD to becoming less noticed with marked reduction in the number of episodes of OCD behaviour. After three weeks Rifaximin was added to the mix of drugs at a dose of 500 mg twice daily and the patients were further observed with a combination of Gentamicin and Rifaximin. Further improvement occurred with vocabulary increase when the patient was next reviewed at three months. There was some lessening in the stool quality but overall the psychotropic improvement was quite marked.

## Claims

1. A formulation, a pharmaceutical or a pharmaceutical preparation comprising at least one active agent, wherein the active agent comprises
rifaximin, extended intestinal release (EIR) rifaximin, rifampicin (or rifampin), rifabutin, rifapentine and rifalazil,
for use in treating, ameliorating and preventing obsessive compulsive disorder (OCD).

2. The formulation, the pharmaceutical or the pharmaceutical preparation for use according to claim 1, wherein the active agent additionally comprises vancomycin or gentamycin.

3. The formulation, the pharmaceutical or the pharmaceutical preparation for use according to claim 1 or 2, wherein the formulation, the pharmaceutical or the pharmaceutical preparation is formulated as a chewable delivery vehicle, a gum, a candy, a lozenge, an ice cream or an ice, or a yogurt.

4. The formulation, the pharmaceutical or the pharmaceutical preparation for use according to claim 1 or 2 wherein
(i) a unit dosage is a pediatric unit dosage, and optionally the unit dosage is between 10 mg and 1100 mg, or
(ii) a unit dosage is 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 or 1100 mg per unit dose.

5. The formulation, the pharmaceutical or the pharmaceutical preparation for use according to claims 1 to 4, wherein a daily dosage is 100, 125, 150, 175, 200, 225, 250, 275, 300, 325, 350, 375, 400, 425, 450, 475, 500, 600, 700, 750, 800, 900, 1000 or 1100 mg per day.

6. The formulation, the pharmaceutical or the pharmaceutical preparation for use according to claim 1 to 5, wherein a unit dosage is set for bid (twice a day), tid (three times a day), four times a day, five times a day or six times a day or more, with the unit dosage and daily dosage adjusted to be: 1000 mg/70 kg a day, or 14 mg/kg a day, for an adult median dose per day (five times a day for adult use, or for a 70 kg individual would be 200 mg per unit dose); or for a pediatric dosage 350 mg/25 kg a day, or 15 to 16 mg/kg, a day; or equivalent (five times a day for pediatric use, or for a 25 kg individual, would be 70 mg per unit dose).

7. The formulation, the pharmaceutical or the pharmaceutical preparation for use according to claims 1 to 6 wherein the formulation, the pharmaceutical or the pharmaceutical preparation further comprises
(i) a flavoring or a sweetening agent, aspartamine, stevia, monk fruit, sucralose, saccharin, cyclamate, xylitol, vanilla, an artificial vanilla or chocolate or strawberry flavor, an artificial chocolate essence, or a mixture or combination thereof; and/or
(ii) a preservative, benzoic acid or potassium sorbate; and/or
(iii) an additive selected from one or more of saline, a defoaming agent, a surfactant agent, a lubricant, an acid neutralizer, a marker, a cell marker, a contrast agent, a dispersal agent, a buffer or a buffering agent, a debittering agent, a pH stabilizer, an acidifying agent, a desweetening agent and/or a coloring agent, vitamin, mineral and/or dietary supplement, or a prebiotic nutrient; and/or
(iv) an additional antimicrobial or antibiotic, wherein optionally the additional antimicrobial or antibiotic comprises: ampicillin, sulbactam, tetracycline, cephalosporin, carbapenem, imipenem, meropenem, monobactam, lincosamide, clindamycin, quinolone, fluoroquinolone, sulphonamide, fradicin, nitroimidazole, metronidazole, tinidazole, anti-clostridial agent, or ramoplanan, aminoglycoside antibiotic, gentamycin, neomycin, streptomycin, paromomycin, verdamicin, mutamicin, sisomicin, netilmicin, retymicin, kanamycin, amphenicol, ansamycin, beta-lactam (β-lactam) antibiotic, carbapenem, cephalosporin, cephamycin, monobactam, oxacephem, lincosamide antibiotic, clindamycin, or lincomycin, glycopeptide antibiotic, vancomycin, teicoplanin, telavancin, bleomycin, ramoplanin, decaplanin, polypeptide antibiotic, actinomycin, actinomycin D, bacitracin, tetracycline, 2,4-diaminopyrimidine class antibiotic, clavacin, clairformin, claviform, clavatin, expansin, gigantin, leucopin, patuline, or a combination thereof.

8. The formulation, the pharmaceutical or the pharmaceutical preparation for use according to claims 1 to 7 wherein the formulation, the pharmaceutical or the pharmaceutical preparation further comprises, or has added to:
(i) at least one probiotic or prebiotic,
wherein optionally the prebiotic comprises inulin, lactulose, extracts of artichoke, chicory root, oats, barley, various legumes, garlic, kale, beans or flacks or an herb, wherein optionally the probiotic comprises a cultured or stool-extracted microorganism or bacteria, or a bacterial component, and optionally the bacteria or bacterial component comprises or is derived from a *Bacteroidetes,* a *Firmicutes,* a *Lactobacilli,* a *Bifidobacteria,* an *E coli* and a *Strep fecalis;*
and/or
(ii) at least one congealing agent, wherein optionally the congealing agent comprises an arrowroot or a plant starch, a powdered flour, a powdered potato or potato starch, an absorbant polymer, an absorbable modified polymer, and/or a corn flour or a corn starch;
and/or
(iii) at least one an anti-inflammatory agent, wherein optionally the anti-inflammatory agent comprises or is 4 or 5-amino-salicylate, olsalazine, mesalazine (also known as mesalamine or 5-aminosalicylic acid (5-ASA)), sulfasalazine and/or balsalazide, or combinations thereof;
and/or
(iv) at least one biofilm disrupting compound, wherein optionally the biofilm disrupting compound comprises an enzyme, a deoxyribonuclease (DNase), N-acetylcysteine, an auranofin, an alginate lyase, glycoside hydrolase dispersin B; a quorum-sensing inhibitor, a ribonucleic acid III inhibiting peptide, *Salvadora persica* extracts, Competence-stimulating peptide, patulin and penicillic acid; peptides - cathelicidin-derived peptides, small lytic peptide, PTP-7, nitric oxide, neo-emulsions; ozone, lytic bacteriophages, lactoferrin, xylitol hydrogel, synthetic iron chelators, cranberry components, curcumin, silver nanoparticles, acetyl-11-keto-β-boswellic acid (AKBA), barley coffee components, probiotics, sinefungin, S-adenosylmethionine, S-adenosyl-homocysteine, *Delisea* furanones, N-sulfonyl homoserine lactones or any combination thereof.

9. The formulation, the pharmaceutical or the pharmaceutical preparation for use according to claims 1 to 8 wherein the formulation, the pharmaceutical or the pharmaceutical preparation is formulated as a delayed or gradual enteric release composition or formulation, and optionally the formulation comprises a gastro-resistant coating designed to dissolve at a pH of 7 in the terminal ileum, e.g., an active ingredient is coated with an acrylic based resin or equivalent, e.g., a poly(meth)acrylate, e.g. a methacrylic acid copolymer B, NF, which dissolves at pH 7 or greater, e.g., comprises a multimatrix (MMX) formulation.

10. The formulation, the pharmaceutical or the pharmaceutical preparation for use according to claims 1 to 9 wherein the formulation, the pharmaceutical or the pharmaceutical preparation is contained in a delivery vehicle, product of manufacture, container, syringe, device or bag.

11. The formulation, the pharmaceutical or the pharmaceutical preparation for use according to claims 1 to 10, wherein the formulation, the pharmaceutical or the pharmaceutical preparation is initially manufactured or formulated as a liquid, a suspension, a gel, a geltab, a semisolid, a tablet, a sachet, a lozenge or a capsule, or as an enteral formulation, or re-formulated for final delivery as a liquid, a suspension, a gel, a geltab, a semisolid, a tablet, a sachet, a lozenge or a capsule, or as an enteral formulation.

## Patentansprüche

1. Eine Formulierung, ein Arzneimittel oder eine pharmazeutische Zubereitung, die mindestens einen Wirkstoff enthält, wobei der Wirkstoff folgende umfasst Rifaximin, Rifaximin mit verlängerter intestinaler Freisetzung (EIR), Rifampicin (oder Rifampin), Rifabutin, Rifapentin und Rifalazil,
zur Verwendung bei der Behandlung, Linderung und Vorbeugung von Zwangsstörungen (OCD = obsessive compulsive disorder).

2. Die Formulierung, das Arzneimittel oder die pharmazeutische Zubereitung zur Verwendung nach Anspruch 1, wobei der Wirkstoff zusätzlich Vancomycin oder Gentamycin umfasst.

3. Die Formulierung, das Arzneimittel oder die pharmazeutische Zubereitung zur Verwendung nach Anspruch 1 oder 2, wobei die Formulierung, das Arzneimittel oder die pharmazeutische Zubereitung als ein kaubares Verabreichungsvehikel, ein Kaugummi, ein Bonbon, eine Lutschtablette, eine Eiscreme oder ein Eis oder ein Joghurt formuliert ist.

4. Die Formulierung, das Arzneimttel oder die pharmazeutische Zubereitung zur Verwendung nach Anspruch 1 oder 2, wobei
(i) eine Dosiseinheit eine pädiatrische Dosiseinheit ist, und optional die Dosiseinheit zwischen 10 mg und 1100 mg liegt, oder
(ii) eine Dosiseinheit 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1000 oder 1100 mg pro Dosiseinheit beträgt.

5. Die Formulierung, das Arzneimittel oder die pharmazeutische Zubereitung zur Verwendung nach den Ansprüchen 1 bis 4, wobei eine Tagesdosis 100, 125, 150, 175, 200, 225, 250, 275, 300, 325, 350, 375, 400, 425, 450, 475, 500, 600, 700, 750, 800, 900, 1000 oder 1100 mg pro Tag beträgt.

6. Die Formulierung, das Arzneimittel oder die pharmazeutische Zubereitung zur Verwendung nach den Ansprüchen 1 bis 5, wobei eine Dosiseinheit für bid (zweimal täglich), tid (dreimal täglich), viermal täglich, fünfmal täglich oder sechsmal täglich oder mehr festgelegt wird, wobei die Dosiseinheit und die Tagesdosis angepasst werden auf: 1000 mg/70 kg pro Tag oder 14 mg/kg pro Tag für eine mediane Erwachsenendosis pro Tag (fünfmal pro Tag für Erwachsene oder für eine 70 kg schwere Person würde 200 mg pro Dosiseinheit betragen); oder für eine pädiatrische Dosis 350 mg/25 kg pro Tag oder 15 bis 16 mg/kg pro Tag; oder äquivalent (fünfmal pro Tag für Kinder oder für eine 25 kg schwere Person würde 70 mg pro Dosiseinheit betragen).

7. Die Formulierung, das Arzneimittel oder die pharmazeutische Zubereitung zur Verwendung nach den Ansprüchen 1 bis 6, wobei die Formulierung, das Arzneimittel oder die pharmazeutische Zubereitung weiterhin umfasst
(i) ein Aroma- oder Süßungsmittel, Aspartamin, Stevia, Mönchsfrucht, Sucralose, Saccharin, Cyclamat, Xylitol, Vanille, ein künstliches Vanille- oder Schokoladen- oder Erdbeeraroma, eine künstliche Schokoladenessenz oder eine Mischung oder Kombination davon; und/oder
(ii) ein Konservierungsmittel, Benzoesäure oder Kaliumsorbat; und/oder
(iii) ein Additiv, ausgewählt aus einem oder mehreren aus Kochsalzlösung, einem Entschäumungsmittel, einem oberflächenaktiven Mittel, einem Gleitmittel, einem Säureneutralisator, einem Marker, einem Zellmarker, einem Kontrastmittel, einem Dispersionsmittel, einem Puffer oder einem Puffermittel, einem Entbitterungsmittel, einem pH-Stabilisator, einem Säuerungsmittel, einem Entsüßungsmittel und/oder einem Färbemittel, einem Vitamin, einem Mineralstoff und/oder einem Nahrungsergänzungsmittel oder einem präbiotischen Nährstoff; und/oder
(iv) ein zusätzliches antimikrobielles Mittel oder Antibiotikum, wobei wahlweise das zusätzliche antimikrobielle Mittel oder Antibiotikum umfasst: Ampicillin, Sulbactam, Tetracyclin, Cephalosporin, Carbapenem, Imipenem, Meropenem, Monobactam, Lincosamid, Clindamycin, Chinolon, Fluorchinolon, Sulfonamid, Fradicin, Nitroimidazol, Metronidazol, Tinidazol, Anti-Clostridienmittel oder Ramoplanan, Aminoglycosid-Antibiotikum, Gentamycin, Neomycin, Streptomycin, Paromomycin, Verdamicin, Mutamicin, Sisomicin, Netilmicin, Re-Tymicin, Kanamycin, Amphenicol, Ansamycin, Beta-Lactam (β-Lactam) Antibiotikum, Carbapenem, Cephalosporin, Cephamycin, Monobactam, Oxacephem, Lincosamid-Antibiotikum, Clindamycin oder Lincomycin, Glycopeptid-Antibiotikum, Vancomycin, Teicoplanin, Telavancin, Bleomycin, Ramoplanin, Decaplanin, Polypeptidantibiotikum, Actinomycin, Actinomycin D, Bacitracin, Tetracyclin, Antibiotikum der 2,4-Diaminopyrimidin-Klasse, Clavacin, Clairformin, Claviform, Clavatin, Expansin, Gigantin, Leukopin, Patulin oder eine Kombination davon.

8. Die Formulierung, das Arzneimittel oder die pharmazeutische Zubereitung zur Verwendung nach den Ansprüchen 1 bis 7, wobei die Formulierung, das Arzneimittel oder die pharmazeutische Zubereitung ferner umfasst oder zugesetzt hat:
(i) mindestens ein Probiotikum oder Präbiotikum,
wobei optional das Präbiotikum Inulin, Lactulose, Extrakte von Artischocke, Zichorienwurzel, Hafer, Gerste, verschiedene Hülsenfrüchte, Knoblauch, Grünkohl, Bohnen oder Flocken oder ein Kraut umfasst,
wobei optional das Probiotikum einen kultivierten oder Stuhlextrahierten Mikroorganismus oder Bakterien oder eine bakterielle Komponente umfasst, und optional die Bakterien oder die bakterielle Komponente einen *Bacteroidetes,* einen *Firmicutes,* einen *Lactobacillen,* einen *Bifidobakterien,* einen *E coli* und einen *Strep fecalis* umfasst oder von diesen abgeleitet ist;
und/oder
(ii) mindestens ein Verdickungsmittel, wobei das Verdickungsmittel optional eine Pfeilwurzel oder eine Pflanzenstärke, ein pulverisiertes Mehl, eine pulverisierte Kartoffel oder Kartoffelstärke, ein absorbierendes Polymer, ein absorbierbares modifiziertes Polymer und/oder ein Maismehl oder eine Maisstärke umfasst;
und/oder
(iii) mindestens ein entzündungshemmendes Mittel, wobei optional das entzündungshemmende Mittel 4 oder 5-Aminosalicylat, Olsalazin, Mesalazin (auch bekannt als Mesalamin oder 5-Aminosalicylsäure (5-ASA)), Sulfasalazin und/oder Balsalazid oder Kombinationen davon umfasst oder ist;
und/oder
(iv) mindestens eine den Biofilm aufbrechende Verbindung, wobei optional die den Biofilm aufbrechende Verbindung ein Enzym, eine Desoxyribonuklease (DNase), N-Acetylcystein, ein Auranofin, eine Alginatlyase, Glykosidhydrolase Dispersin B; einen Quorum-sensing Inhibitor, ein Ribonukleinsäure-IIIinhibierendes Peptid, *Salvadora persica*-Extrakte, ein kompetenzstimulierendes Peptid, Patulin und Penicillinsäure; Peptide - von Cathelicidin abgeleitete Peptide, kleines lytisches Peptid, PTP-7, Stickstoffmonoxid, Neo-Emulsionen; Ozon, lytische Bakteriophagen, Lactoferrin, Xylitol-Hydrogel, synthetische Eisenchelatbildner, Cranberry-Komponenten, Curcumin, Silbernanopartikel, Acetyl-11-keto-β-Boswelliasäure (AKBA), Gerstenkaffee-Komponenten, Probiotika, Sinefungin, S-Adenosylmethionin, S-Adenosyl-Homocystein, *Delisea-Furanone,* N-Sulfonyl-Homoserin-Lactone oder jede Kombination davon umfasst.

9. Die Formulierung, das Arzneimittel oder die pharmazeutische Zubereitung zur Verwendung nach den Ansprüchen 1 bis 8, wobei die Formulierung, das Arzneimittel oder die pharmazeutische Zubereitung als Zusammensetzung oder Formulierung mit verzögerter oder allmählicher magensaftresistenter Freisetzung formuliert ist und optional die Formulierung eine gastro-resistente Beschichtung umfasst, die so ausgelegt ist, dass sie sich bei einem pH-Wert von 7 im terminalen Ileum auflöst, z.B, ein Wirkstoff mit einem Harz auf Acrylbasis oder einem Äquivalent, z.B. einem Poly(meth)acrylat, z.B. einem Methacrylsäure-Copolymer B, NF, beschichtet ist, das sich bei einem pH-Wert von 7 oder höher auflöst, z.B. eine Multimatrix (MMX)-Formulierung umfasst.

10. Die Formulierung, das Arzneimittel oder die pharmazeutische Zubereitung zur Verwendung nach den Ansprüchen 1 bis 9, wobei die Formulierung, das Arzneimittel oder die pharmazeutische Zubereitung in einem Verabreichungsvehikel, einem Herstellungsprodukt, einem Behälter, einer Spritze, einer Vorrichtung oder einem Beutel enthalten ist.

11. Die Formulierung, das Arzneimittel oder die pharmazeutische Zubereitung zur Verwendung nach den Ansprüchen 1 bis 10, wobei die Formulierung, das Arzneimittel oder die pharmazeutische Zubereitung anfänglich als eine Flüssigkeit, eine Suspension, ein Gel, ein Geltab, ein Halbfeststoff, eine Tablette, ein Beutel, eine Lutschtablette oder eine Kapsel oder als eine enterale Formulierung hergestellt oder formuliert wird oder zur endgültigen Verabreichung als eine Flüssigkeit, eine Suspension, ein Gel, ein Geltab, ein Halbfeststoff, eine Tablette, ein Beutel, eine Lutschtablette oder eine Kapsel oder als eine enterale Formulierung umformuliert wird.

## Revendications

1. Formulation, produit pharmaceutique ou préparation pharmaceutique comprenant au moins un agent actif, dans lequel/laquelle l'agent actif comprend
la rifaximine, la rifaximine à libération intestinale prolongée (EIR), la rifampicine (ou rifampine), la rifabutine, la rifapentine et le rifalazil, destiné(e) à être utilisé(e) dans le traitement, l'atténuation et la prévention d'un trouble obsessionnel compulsif (TOC).

2. Formulation, produit pharmaceutique ou préparation pharmaceutique destiné(e) à être utilisé(e) selon la revendication 1, dans lequel/laquelle l'agent actif comprend en outre la vancomycine ou la gentamycine.

3. Formulation, produit pharmaceutique ou préparation pharmaceutique destiné(e) à être utilisé(e) selon la revendication 1 ou 2, dans lequel/laquelle la formulation, le produit pharmaceutique ou la préparation pharmaceutique est formulé(e) sous forme d'un véhicule de délivrance à mâcher, d'une gomme, d'un bonbon, d'une pastille, d'une crème glacée ou d'une glace, ou d'un yaourt.

4. Formulation, produit pharmaceutique ou préparation pharmaceutique destiné(e) à être utilisé(e) selon la revendication 1 ou 2, dans lequel/laquelle
(i) une unité de prise est une unité de prise pédiatrique, et en option l'unité de prise est comprise entre 10 mg et 1 100 mg, ou
(ii) une unité de prise est de 10, 20, 30, 40, 50, 60, 70, 75, 80, 90, 100, 125, 150, 175, 200, 225, 250, 275, 300, 325, 350, 375, 400, 425, 450, 475, 500, 600, 700, 750, 800, 900, 1 000 ou 1 100 mg par dose unitaire.

5. Formulation, produit pharmaceutique ou préparation pharmaceutique destiné(e) à être utilisé(e) selon les revendications 1 à 4, dans lequel/laquelle une posologie journalière est de 100, 125, 150, 175, 200, 225, 250, 275, 300, 325, 350, 375, 400, 425, 450, 475, 500, 600, 700, 750, 800, 900, 1 000 ou 1 100 mg par jour.

6. Formulation, produit pharmaceutique ou préparation pharmaceutique destiné(e) à être utilisé(e) selon l'une quelconque des revendications 1 à 5, dans lequel/laquelle une unité de prise est établie pour BID (deux fois par jour), TID (trois fois par jour), quatre fois par jour, cinq fois par jour, ou six fois par jour ou plus, la dose unitaire et la posologie journalière étant ajustées pour être : 1 000 mg/70 kg par jour, soit 14 mg/kg par jour, pour une dose médiane pour un adulte par jour (cinq fois par jour pour l'emploi chez l'adulte, soit 200 mg par dose unitaire pour un individu de 70 kg) ; ou pour une posologie pédiatrique 350 mg/25 kg par jour, soit 15 à 16 mg/kg par jour ; ou équivalent (cinq fois par jour pour l'emploi pédiatrique, soit 70 mg par dose unitaire pour un individu de 25 kg).

7. Formulation, produit pharmaceutique ou préparation pharmaceutique destiné(e) à être utilisé(e) selon les revendications 1 à 6, dans lequel/laquelle la formulation, le produit pharmaceutique ou la préparation pharmaceutique comprend en outre
(i) un agent aromatisant ou un agent édulcorant, de l'aspartamine, de la stévia, du fruit du moine, du sucralose, de la saccharine, du cyclamate, du xylitol, de la vanille, un arôme artificiel vanille ou chocolat ou fraise, une essence artificielle de chocolat, ou un mélange ou une association de ceux-ci ; et/ou
(ii) un conservateur, l'acide benzoïque ou le sorbate de potassium ; et/ou
(iii) un additif choisi parmi un ou plusieurs consistant en une solution salée, un agent antimousse, un agent tensioactif, un lubrifiant, un agent neutralisant les acides, un marqueur, un marqueur cellulaire, un agent de contraste, un agent dispersant, un tampon ou un agent tamponnant, un agent désamérisant, un stabilisant du pH, un agent acidifiant, un agent désédulcorant et/ou un agent colorant, une vitamine, un complément alimentaire et/ou minéral, ou un nutriment prébiotique ; et/ou
(iv) un antibiotique ou antimicrobien supplémentaire, en option l'antibiotique ou antimicrobien supplémentaire comprenant : l'ampicilline, le sulbactame, la tétracycline, une céphalosporine, un carbapénème, un imipénème, un méropénème, un monobactame, le lincosamide, la clindamycine, une quinolone, une fluoroquinolone, un sulfonamide, la fradicine, le nitroimidazole, le métronidazole, le tinidazole, un agent anti-Clostridium, or le ramoplanan, un antibiotique aminoglycoside, la gentamycine, la néomycine, la streptomycine, la paromomycine, la verdamicine, la mutamicine, la sisomicine, la nétilmicine, la rétymicine, la kanamycine, l'amphénicol, l'ansamycine, un antibiotique bêta-lactame (β-lactame), un carbapénème, une céphalosporine, la céphamycine, un monobactame, un oxacéphème, un antibiotique lincosamide, la clindamycine, ou la lincomycine, un antibiotique glycopeptidique, la vancomycine, la teicoplanine, la télavancine, la bléomycine, la ramoplanine, la décaplanine, un antibiotique polypeptidique, l'actinomycine, l'actinomycine D, la bacitracine, la tétracycline, un antibiotique de la classe des 2,4-diaminopyrimidines, la clavacine, la clairformine, le claviforme, la clavatine, l'expansine, la gigantine, la leucopine, la patuline, ou une association de tels antimicrobiens ou antibiotiques.

8. Formulation, produit pharmaceutique ou préparation pharmaceutique destiné(e) à être utilisé(e) selon les revendications 1 à 7, dans lequel/laquelle la formulation, le produit pharmaceutique ou la préparation pharmaceutique en outre comprend, ou auquel/à laquelle est ajouté :
(i) au moins un probiotique ou prébiotique,
où en option le prébiotique comprend l'inuline, le lactulose, des extraits d'artichaut, de racine de chicorée, d'avoine, d'orge, de divers légumes, d'ail, de chou frisé, de haricots ou de lin ou d'une herbe aromatique,
où en option le probiotique comprend un micro-organisme ou une bactérie, cultivé(e) ou extrait(e) de fèces, ou un composant bactérien, et en option la bactérie ou le composant bactérien comprend ou est issu d'une *Bacteroidetes,* une *Firmicutes,* un *Lactobacillus,* un *Bifidobacterium,* une *E coli* et un *Streptococcus fecalis ;*
et/ou
(ii) au moins un agent de congélation, où en option l'agent de congélation comprend un amidon végétal ou un amidon de marante, une farine en poudre, une poudre de pomme de terre ou de la fécule de pomme de terre, un polymère absorbant, un polymère modifié absorbable, et/ou une farine de maïs ou un amidon de maïs ;
et/ou
(iii) au moins un agent anti-inflammatoire, où en option l'agent anti-inflammatoire comprend ou est le 4 ou 5-amino-salicylate, l'olsalazine, la mésalazine (également connue sous la dénomination de mésalamine ou acide 5-aminosalicylique (5-ASA)), la sulfasalazine et/ou le balsalazide, ou des associations de tels agents ;
et/ou
(iv) au moins un composé détruisant les biofilms, où en option le composé détruisant les biofilms comprend une enzyme, une désoxyribonucléase (DNase), la N-acétylcystéine, une auranofine, une alginate lyase, une glycoside hydrolase dispersine B ; un inhibiteur de la détection du quorum, un peptide inhibant l'acide ribonucléique III, des extraits de *Salvadora persica*, un peptide déclencheur de la compétence, la patuline et l'acide pénicillique ; des peptides - des peptides dérivés de la cathélicidine, le petit peptide lytique, PTP-7, l'oxyde nitrique, les néoémulsions ; l'ozone, les bactériophages lytiques, la lactoferrine, l'hydrogel de xylitol, les chélateurs de fer synthétiques, les composants d'airelle, la curcumine, des nanoparticules d'argent, l'acide acétyl-11-céto-β-boswellique (AKBA), des composants du café d'orge, des probiotiques, la sinéfungine, la S-adénosylméthionine, la S-adénosyl-homocystéine, les furanones de *Delisea,* les N-sulfonyl homosérine lactones ou une association quelconque de tels composés.

9. Formulation, produit pharmaceutique ou préparation pharmaceutique destiné(e) à être utilisé(e) selon les revendications 1 à 8, dans lequel/laquelle la formulation, le produit pharmaceutique ou la préparation pharmaceutique est formulé(e) sous forme d'une formulation ou composition à libération entérique retardée ou progressive, et en option la formulation comprend un enrobage gastro-résistant conçu pour se dissoudre à un pH de 7 dans l'iléon terminal, par exemple un composant actif est enrobé avec une résine à base acrylique ou équivalente, par exemple un poly(méth)acrylate, par exemple un copolymère d'acide méthacrylique B, NF, qui se dissout à pH 7 ou à un pH plus élevé, par exemple comprend une formulation multimatrice (MMX).

10. Formulation, produit pharmaceutique ou préparation pharmaceutique destiné(e) à être utilisé(e) selon les revendications 1 à 9, dans lequel/laquelle la formulation, le produit pharmaceutique ou la préparation pharmaceutique est contenu(e) dans un véhicule de délivrance, un produit de fabrication, un contenant, une seringue, un dispositif ou une poche.

11. Formulation, produit pharmaceutique ou préparation pharmaceutique destiné(e) à être utilisé(e) selon les revendications 1 à 10, dans lequel/laquelle la formulation, le produit pharmaceutique ou la préparation pharmaceutique est initialement fabriqué(e) ou formulé(e) sous forme d'un liquide, d'une suspension, d'un gel, d'un comprimé de gel, d'un semi-solide, d'un comprimé, d'un sachet, d'une pastille ou d'une capsule, ou sous forme d'une formulation entérique, ou reformulé(e) pour délivrance finale sous forme d'un liquide, d'une suspension, d'un gel, d'un comprimé de gel, d'un semi-solide, d'un comprimé, d'un sachet, d'une pastille ou d'une capsule, ou sous forme d'une formulation entérique.
